# EUROPEAN PATENT APPLICATION

(11) **EP 2 602 331 A1**
(43) Date of publication of application: **12.06.2013**
(21) Application number: 11192704.2
(22) Date of filing: 09.12.2011
(51) Int. Cl.: C12Q 1/68

(54) **Diagnostic reagent embedded in wax as an internal standard for nucleic acid preparation or nucleic acid detection**

(71) Applicant: Qiagen GmbH, 40724 Hilden (DE)
(72) Inventor: Himmelreich, Ralf Dr., 40764 Langenfeld (DE); Holzer, Eva, 40724 Hilden (DE); Jeziorski, Markus, 40764 Langenfeld (DE)
(74) Representative: Gille Hrabal

(57) **Abstract**

The present invention relates to a diagnostic reagent embedded in a waxy substance, to a method for production thereof, and to use thereof as an internal standard in methods for nucleic acid preparation and/or nucleic acid detection. The invention further relates to a kit, comprising the diagnostic reagent.

The diagnostic reagent for methods of nucleic acid preparation and/or nucleic acid detection comprises at least one microorganism and/or purified nucleic acid, wherein the microorganism and/or nucleic acid is embedded in a substance, preferably a waxy substance with hydrophobic properties. The diagnostic reagent is adapted to release the embedded microorganism and/or nucleic acid depending on the ambient temperature.

## Description

The present invention relates to a diagnostic reagent embedded in wax, to a method for production thereof, and to use thereof as an internal standard in methods for nucleic acid preparation and/or nucleic acid detection. The invention further relates to a kit, comprising the diagnostic reagent.

Detection of infectious bioparticles, such as microorganisms, for example bacteria, viruses, fungi, protozoa, or the like, in samples of different origin is increasingly important. For example, when selecting an appropriate treatment method or therapeutic agent for patients with infections, it is critical to determine which pathogenic agent is present. The incubation of samples, such as blood or urine for this purpose often necessitates lengthy tests with various antibiotics to identify which pathogens are present and which therapeutic agents would be appropriate for the treatment. Detection of bioparticles in samples such as food, water, or the like for purpose of quality control is also increasingly important.

There have been efforts to develop faster and more reliable diagnostic methods which enable the rapid identification of pathogens. A common method is to release the nucleic acids of the pathogens present in a sample, specifically amplifying these nucleic acids, and then detecting them. For this purpose, it is necessary to collect sample material (e.g., blood, sputum, urine, feces, cerebrospinal fluid, swab samples from oral/nasal/genital cavities), perform lysis to break down the cells, prepare the nucleic acid, amplify the nucleic acid and eventually detect the amplified nucleic acid.

The amplification of nucleic acids is usually done by polymerase chain reaction (PCR) or other amplification methods such as ligase chain reaction (LCR), gap-filling LCR (Gap-LCR), nucleic acid sequence-based amplification (NASBA), loop mediated isothermal amplification (LAMP) and transcription-mediated amplification (TMA).

The methods for nucleic acid preparation and nucleic acid amplification as well as nucleic acid detection can be carried out on a laboratory scale, i.e., on a microliter or millilitre scale. Microfluidic methods have also been developed recently. In microfluidic methods small liquid amounts which have a lower volume, by a few orders of magnitude, than normal liquid drops are handled and processed. Devices for carrying out microfluidic methods are also referred to as lab-on-a-chip (LoC), i.e., a single-use consumable in the form of a chip having approximately the size of a credit card. The use of such microfluidic devices usually demands minimal time and effort, and no special training or education is required to carry out the analysis.

An essential problem in preparing and amplifying nucleic acid, both on a laboratory and microfluidic scale, is quality control in order to gain valid and therefore conclusive results. A special problem in this regard is the false-negative result, wherein a target nucleic acid is present in the sample but not detected due to errors in the workflow or reaction conditions and/or setup. The lysis of the cells, the isolation of the nucleic acids and the amplification may be prone to errors.

It is therefore known (WO 02/052041, WO 2005/04762) to add internal control substances to the PCR, which are designed to confirm the correct conducting of the amplification. The internal standard may be added to the sample before the first processing step, for example before the purification or the lysis, or even before the PCR. Internal control standards may be purified nucleic acids, protein-complexed nucleic acids (armoured RNA) or inactive virus standards.

The internal standards resembled by free nucleic acids are still not satisfactory in that they are generally instable products which are not storable under standard conditions.

Therefore, there is a need for accurate, fast and reliable internal standards which allow the quality control of a nucleic acid preparation and/or nucleic acid detection in a simple manner. It should also be considered that the internal standard can be easily carried out on a laboratory and microfluidic scale. An object of the invention is to provide an improved diagnostic reagent, a method for production thereof and a method for use thereof.

The object of the invention is achieved by the diagnostic reagent according to claim 1. Further embodiments of the invention are recited in the dependent claims.

The diagnostic reagent for methods of nucleic acid preparation and/or nucleic acid detection comprises at least one microorganism and/or purified nucleic acid, wherein the microorganism and/or nucleic acid is/are embedded in a substance. The preferred substance for embedding is a waxy substance especially with hydrophobic properties. The diagnostic reagent is adapted to release the embedded microorganism and/or nucleic acid depending on the temperature, particularly the surrounding temperature.

Microorganisms within the meaning of the invention are bacteria, viruses, fungi, bacteriophages, yeasts, spores, parasites, plant cells, animal or human cells, gametes, plasmids, and viroids. Examples for suitable microorganisms include bacteria such as Corynebacterium glutamicum, bacteriophages such as Lambda, T7, fr, qβ and M13, viruses such as TMV, as well as single-cell organisms (protozoas) such as ciliate, ciliophora (Paramecium caudatum), rotatoria, rotifer (Philodina roseola and Habrotrocha constricta). The microorganisms comprise genetic material in the form of nucleic acids. In an embodiment, the diagnostic reagent comprises purified nucleic acids. The purified nucleic acid may optionally be enriched with a stabilizer such as polyamine. Usually, the diagnostic reagent comprises one type of microorganism, but it is also possible that multiple types are present, for example two or more types of bacteria. A combination of one bacterium type and one virus type or one bacterium type and one bacteriophage is also possible. It is particularly possible to include nucleic acids from different sources having additionally different concentrations, which may build a kind of "analytical window".

The waxy substance for entrapping the microorganism is essentially water insoluble and solid under standard conditions. When the waxy substance is heated to a critical temperature, it begins to melt and form a dispersible liquid. The waxy substance may be any naturally occurring or synthetic wax with a desired melting temperature. A preferred waxy substance is paraffin wax with a melting point between 46° and 68° C. Other suitable waxy substances include polyethylene glycol (PEG), also known under the tradename Carbowax. Depending on the desired properties, such as melting point, inertness, solubility, buoyancy, etc., several waxy substances may be combined in various proportions to achieve the desired results.

When the diagnostic reagent is preferably heated to a melting temperature of the waxy substance, the embedded microorganism and/or nucleic acid is/are released into the surrounding medium and can function as an internal standard. The melting temperature of the waxy substance is preferably selected so that during the preparation of the nucleic acid, for example, during an enzymatic lysis step which is usually conducted at a temperature of about 56° - 70° C, the waxy substance is melted and the constituents of the diagnostic reagents are released into the liquid phase of a lysate. The diagnostic reagent may be processed along with the clinical sample or specimen. The isolated nucleic acids of the internal standard can be detected by PCR-based methods and can thereby indicate whether the preparation conditions as well as the PCR itself are valid for conclusive diagnostic results.

The diagnostic reagent according to the invention has the advantage that the constituents of the diagnostic reagent are embedded in a waxy substance and therefore protected against outside influences. Usually the diagnostic reagent is added to a lysis buffer or an enzymatic reaction buffer, wherein the diagnostic reagent is separated from the other medium by the waxy substance with hydrophobic properties. This ensures that basically no reaction between the diagnostic reagent and the ambient medium such as a lysis buffer or reaction buffer occurs as long as the waxy substance encapsulates the constituents of the diagnostic reagent under standard conditions. The microorganism and/or purified nucleic acid embedded in the waxy substance is/are thereby protected against degradation and ageing, which for example can be caused by the substances of the lysis buffer. In this way, a long-term storage of the diagnostic reagent is enabled, It is furthermore possible to store the diagnostic reagent in a lysis buffer containing a detergent. Thus, the reliability and function of the diagnostic reagent as an internal standard for nucleic acid preparation and/or nucleic acid detection is enhanced compared to the above-mentioned prior art.

The heat-releasable diagnostic reagent is further advantageous with regard to a precise and consistent release. By adapting the melting temperature of the waxy substance to certain process temperatures, for example temperatures of the nucleic acid preparation and/or nucleic acid detection, the release of the diagnostic reagent can be precisely controlled. In this way, the PCR reaction can be improved by avoiding polymerization of unspecific side products. The specificity, yield, and precision of the PCR can be increased. This also entails, that mispriming can be reduced. Although the primers in PCR are usually designed to amplify specific sections of a DNA sequence, amplifications of other sections can occur when a primer binds to an unintended template (mispriming). Mispriming generally occurs due to poorly optimised conditions. The heat-releasable diagnostic reagent leads to optimised conditions increasing the amplification efficiency and specificity.

Preferably the diagnostic reagent is provided as a solid or at least on the surface solid composition under standard conditions. Solid means that the composition of the diagnostic reagent is dimensionally stable under standard conditions. Standard conditions within the meaning of the invention refer to an ambient temperature of about 15-25° C and/or an ambient air pressure of about 1 bar.

The analyte of a nucleic acid preparation and/or nucleic acid detection method is usually provided in liquid form, whereby the diagnostic reagent according to prior art is also added in liquid form in practice. The liquid form of the diagnostic reagent is assumed to represent a standard for reproducible results. However, it was surprisingly found that the diagnostic reagent provided in solid form under standard conditions can also validate results in a reliable and reproducible manner.

The solid composition of the diagnostic reagent facilitates the handling and dosage. The solid composition of the diagnostic reagent can be easily handled, since the user can simply add the solid composition to the sample to be analyzed. As the dosing of the diagnostic reagent does not require any pipetting, the related work and effort can be minimized. Furthermore, the likelihood of errors can be reduced, since it is easier to control the presence of a diagnostic reagent which is provided in a solid form. In a further aspect, contact with the microorganisms present in the diagnostic reagent, loss of material and the risk of contamination which to some extent occur inevitably when using suspensions or liquids, can be avoided at the same time.

In a preferred embodiment of the invention the diagnostic reagent further comprises an emulsifier. The diagnostic reagent is preferably provided as a solid emulsion under standard conditions. In the emulsion the at least one microorganism or the nucleic acid is preferably evenly dispersed throughout the embedding waxy substance. When the diagnostic reagent comprises further constituents, the further constituents are preferably similarly dispersed throughout the embedding waxy substance. The emulsifier stabilizes the emulsion and facilitates the embedding of the at least one microorganism or the nucleic acid especially during production thereof. The emulsifier allows for a homogeneous distribution of the microorganism(s) or the nucleic acids in the waxy substance which in turn ensures that the obtained diagnostic reagent is scalable in a reproducible way.

The emulsifier is preferably added to the diagnostic reagent during production of a liquid emulsion comprising a molten waxy substance and the at least one microorganism or the nucleic acid. Afterwards, the liquid emulsion may be solidified to a solid emulsion under standard conditions. Optionally, further tempering devices for cooling may be employed to control the process continuously. Preferably the amount of emulsifier in relation to the total amount of the diagnostic reagent is at least 3 %. The emulsifier is particularly selected to be compatible with the PCR reaction. Suitable emulsifiers are, for example, polysorbate 20 (Tween 20) or polyethoxyethanols (Nonident P40; IGEPAL)

The diagnostic reagent may further comprise excipients which are suitable for producing the diagnostic reagent with particular properties. Suitable excipients include binders and/or fillers such as polyethyleneglycols, polypyrolidones and Polyvinylalcohols.

During the production of the diagnostic reagent, one or more buffers may be added to the reagent. Preferably use is made of buffers which have stabilizing and/or lysing properties for microorganisms. Suitable buffers are TE bufferand PBS. In an embodiment, the diagnostic reagent further comprises a chelating agent such as EDTA or EGTA.

The diagnostic reagent according to the invention may further comprise appropriate hydrophobic or hydrophilic dyes in order to provide color coding means. This means that the diagnostic reagents may be differently coloured depending on the microorganism embedded therein, making it possible to easily distinguish between them. The dyes may be added as solids or in the form of a solution, for example, water and/or glycerol and/or customary alcohols, to produce the diagnostic reagent according to the invention.

In an embodiment, the diagnostic reagent is colored by its wax proportion using a hydrophobic dye like Solvent Blue or Solvent Red to easily distinguish between different diagnostic reagents.

In a preferred embodiment of the invention the diagnostic reagent comprises an intercalating dye. The intercalating dye can bind to doublestranded DNA leading to an increase of fluorescence, thereby enabling qualitative and quantitative data from PCR reactions. As the fluorescence signal increases proportionally to the amount of amplification, the intercalating dye in the diagnostic reagent particularly has a beneficial effect for the quantitative PCR.

In another embodiment of the invention the diagnostic reagent may further comprise one or more enzymes in order to facilitate the lysis. For this purpose, proteases such as proteinase K or subtilins may be considered. Other suitable enzymes include lipases, lysozyme, lyticase, zymolase, DNA polymerase (Taq polymerase), GST polymerase, thermostable recombination enzymes (RecA homolog, helicase) as well as thermostable nuclease (endonuclease, exonuclease, nicking endonuclease).

The enzymes and their temperature-controlled release are particularly advantageous for the biochemical reaction to the extent that either the specificity of the PCR or the nucleic acid amplification can be improved or that enzymatic reactions may be carried out in a serial manner using different temperatures. As an example of an enzymatic reaction in a serial manner, the lysis of bacteria may be done using lysozyme at a temperature of 37° C, followed by a lysis with proteinase K at a temperature of 56° - 60° C. The specificity of the PCR can likewise be increased by magnesium or nucleotide.

The diagnostic reagent according to the invention may further comprise particles made of hard material, such as glass, silicon carbide, or zirconium carbide. Magnetic silica particles or anion exchange resin particles are also suited for this purpose. The preferred diameter of these hard particles is 100 to 800 nm. Hard particles are particularly advantageous for reagents comprising microorganisms which are difficult to disrupt. The lysis can, for example, be carried out by using Vortex instruments or bead mills. The hard particles facilitate the lysis of the microorganisms through mechanical disruption. The hard particles may amount to 10 to 20 mg, preferably about 14 to 17 mg, for 1.5 ml of bacterial culture liquid. Depending on the application, glass particles with a different diameter may be used.

The diagnostic reagent may be produced by melting the waxy substance, preferably at a temperature substantially above the melting temperature of the waxy substance and mixing the melted waxy substance with the at least one microorganism in order to produce a preferably homogeneous emulsion. The microorganism to be embedded may be preheated to minimize temperature deviations in the emulsion. Afterwards the obtained emulsion is solidified under standard conditions to form a solid composition.

In an embodiment, the diagnostic reagent is provided in the form of pellets or sticks under standard conditions. For this, the solidification is preferably done by using aliquots of the emulsion dispensed into appropriate molds to produce the diagnostic reagents in form of pellets, sticks or other shapes. The shape of pellets or sticks allows for an easy and fast delivery of the diagnostic reagent in desired amounts. Alternatively, the liquid emulsion may be extruded or sprayed into particles or beads. The obtained particles or beads likewise enable an easy dosage of the diagnostic reagent.

The diagnostic reagent according to the invention may be used as an internal standard for nucleic acid preparation and/or nucleic acid detection, for example a disruption or lysis method. For this purpose, the sample may be mixed with the diagnostic reagent and an appropriate lysis buffer. Afterwards the mixture may be treated by means of heat, sonication, or bead beating.

In an embodiment, the microorganisms and/or nucleic acids of the diagnostic reagent such as bacteria or viruses may be adapted to retain the cell wall and/or morphology during production of the diagnostic reagent. After releasing the microorganisms and/or nucleic acids, for example in a lysis buffer, the cell wall and/or morphology prevents or reduces the exposure of the microorganisms and/or nucleic acids.

In an embodiment, the microorganism and/or nucleic acid of the diagnostic reagent comprises nucleic acid sequences found in the genetic information such as the DNA/RNA of pathogens. The diagnostic reagent may comprise an infectious agent. By embedding the infectious agent, for example nucleic acid sequences found in the DNA/RNA of pathogen, in a substance, particularly in a waxy substance, the level of infectiousness can be reduced, substantially inactivating the infectivity of such a diagnostic reagent. Furthermore, the release of the infectious constituents of the diagnostic reagent into the environment can be prevented. After releasing the embedded microorganism and/or nucleic acid, for example by heating above a critical temperature of a waxy substance, the diagnostic reagent becomes effective for nucleic acid preparation and/or nucleic acid detection. By embedding an infectious agent especially in a waxy substance that is adapted to release the embedded constituents depending on the surrounding temperature, the infectivity of such a diagnostic reagent can be substantially eliminated.

The kit according to the invention includes the diagnostic reagent and all solvents and/or reagents required for carrying out the desired reactions for nucleic acid preparation and/or nucleic acid detection. The kit improves the reliability of the nucleic acid preparation and/or detection method as the chance of neglecting the quality control procedure is minimized. In an embodiment of the invention the kit further comprises a microfluidic device.

In a further embodiment of the invention, the waxy substance is provided as a solid enclosure under standard conditions for containing the constituents of the diagnostic reagent such as microorganisms in liquid form. The at least one microorganism and/or purified nucleic acid is/are thereby embedded by a waxy substance in form of a solid enclosure. The preferred solid enclosure is provided as a capsule wherein the waxy substance forms the solid shell under standard conditions. In another embodiment of the invention the solid enclosure features a sealable hole for filling the enclosure with the constituents of the diagnostic reagent such as the microorganism(s). A plug or a drop of waxy substance may be inserted into the filling hole to seal the solid enclosure. By heating the solid enclosure such as a capsule to the melting temperature of the waxy substance, the constituents of the diagnostic reagent are released into the ambient medium and can function as an internal standard. The solid enclosure of the diagnostic reagent provides an improved protection against outside influences. The waxy substance forms a solid shell for the contained microorganism and/or purified nucleic acid, thereby functioning as a sealing means. The solid enclosure under standard conditions provides a complete protection of the contained substances and increasing the long-term stability of the diagnostic reagent. This embodiment is particularly advantageous for sensitive or highly reactive constituents of the diagnostic reagent.

In an embodiment, the solid enclosure under standard conditions is provided as a hollow tube or sleeve. The hollow tube or sleeve preferably contains the at least one microorganism and/or purified nucleic acid in liquid form. To seal the tube or sleeve the openings may be sealed with a drop of wax or a plug. The hollow tube or sleeve likewise provides a complete protection of the diagnostic reagent.

The following examples describe the invention in more detail.

### Example 1: Production and test of nucleic acid emulsion embedded in wax

In this example, paraffin wax (melting point 53° - 57° C) was melted, dyed with solvent blue 35 and tempered to 70° - 80° C. A wax volume of 1 0 ml was transferred to an appropriate syringe. The outlet of the syringe was closed with a blind plug, and stored temporarily at 80° C in a water bath. 500 *µ*l of *E.coli* genomic DNA (gDNA) solution (one part of the gDNA solution diluted with TE buffer to 1 0 ng/*µ*l, one part with 10% Tween 20) was added to the syringe and vortexed thoroughly. The blind plug was removed and a flexible silicone tube (Versilic silicon tube, 3 x 5 mm, code: 760210) which had been slitted was attached on the outlet of the syringe. The flexible tube was filled with the wax emulsion and cooled under standard conditions to about 20° C, whereby the emulsion solidified. The solid emulsion was withdrawn from the flexible tube, washed with water, dried again for approximately 20 minutes and stored in a tube with screw cap. The obtained diagnostic reagent in form of a wax emulsion stick can be used in various amounts for extraction/PCR reaction.

The solidified wax emulsion stick can be melted by heating it to the melting temperature of the wax, whereby the at least one microorganism embedded in the wax is released. The release can for example be done by melting the wax emulsion stick inside a test tube and centrifuging it. The released microorganism which was formerly included in the wax emulsion stick is then visible as a transparent aqueous phase at the bottom of the test tube.

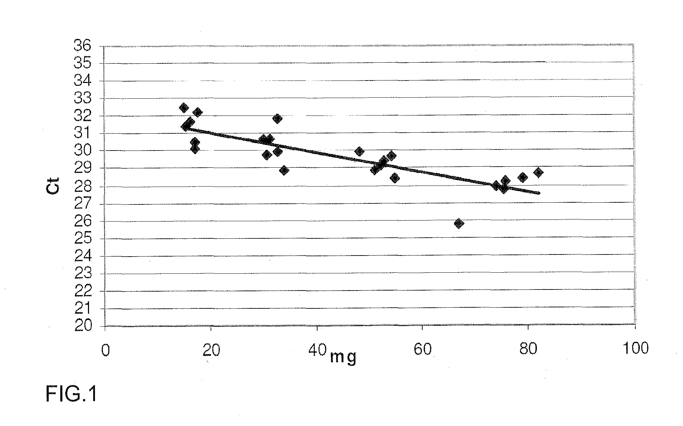

The PCR detection was carried out by pipetting 200*µ*l TE buffer into a 1,5 ml micro test tube, adding a portion of the wax emulsion stick (3 - 15 mm corresponding to 15 - 75 mg) and incubating the micro test tube for 5 min at 95 ° C in a thermomixer with a mixing frequency of 400 rpm. Then, the micro test tube was cooled to room temperature and 5 *µ*l were used in quantitative PCR (25*µ*l). The results of the quantification obtained in example 1 are shown in Fig. 1. The Ct value is the amount of detected nucleic acid. The diagnostic reagent is applied using a portion of the wax emulsion stick. The Ct value is depicted as a function of the amount of applied diagnostic reagent in mg. The coefficient of determination (R²) is 0,6695. As is apparent from the regression line, there is a substantially linear correlation between the applied amount of diagnostic reagent and the Ct value.

### Example 2: Production and test of E.coli cell suspension embedded in wax

In this example, paraffin wax (melting point 53° - 57° C) was melted, dyed with solvent blue 35 and tempered to 70° - 80° C. A wax volume of 1,8 ml was transferred to an appropriate syringe, the outlet of the syringe was closed with a blind plug, and stored temporarily at 80° C in a water bath. 200 *µ*l of *E.coli* cell suspension (one part overnight bacterial culture diluted with a ratio of 1 :10, one part TE buffer + isopropanol abs.) was added to the syringe and vortexed thoroughly. The blind plug was removed and a flexible silicone tube (Versilic silicon tube, 3 x 5 mm, code: 760210) which had been slitted was attached on the outlet of the syringe. The flexible tube was filled with the wax emulsion and cooled under standard conditions, whereby the emulsion solidified. The solid emulsion was withdrawn from the flexible tube, washed with water, dried and stored in a tube with screw cap. The obtained diagnostic reagent in form of a wax emulsion stick can be used in various amounts for extraction/PCR detection.

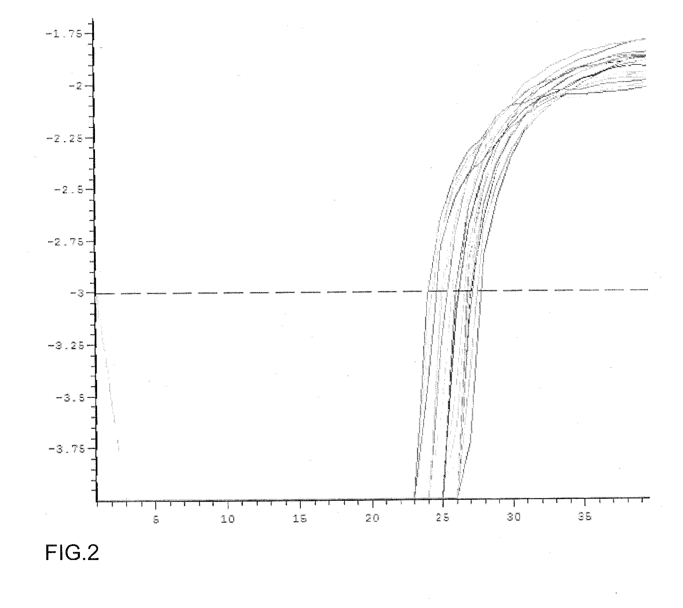

The extraction and PCR detection was carried out by pipetting 200 *µ*l TE buffer into a 1,5 ml micro test tube, adding a portion of the diagnostic reagent (3 - 15 mm corresponding to 15 - 75 mg) and incubating the micro test tube for 5 min at 95 ° C in a thermomixer with a mixing frequency of 400 rpm. Then, the micro test tube was cooled to room temperature and 5 *µ*l were used in quantitative PCR (25*µ*l). The results obtained in example 2 are shown in Fig. 2. The figure shows the amplification plots using real-time PCR for quantitative measurement. The fluorescence signal in logarithmic scale (y-axis) is plotted against the PCR cycle number (x-axis). The threshold line (horizontal broken line) illustrates the point at which a reaction reaches a fluorescent intensity above background. The amplification plots demonstrate the success of the lysis and extraction of the E.coli cells.

### Example 3: Production of wax capsules

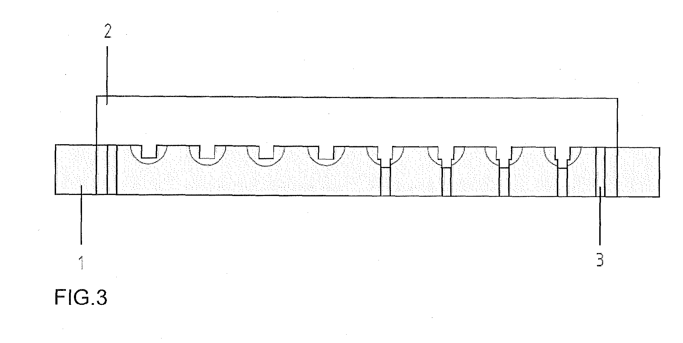

In this example, the wax capsules were produced using two negative molds which shape on one side the outer form of the hemispheres and on the other side the inner form. The first negative mold was made from silicone using a positive mold produced by rapid prototyping on an OBJET Eden250 3D printing system. The silicone (PDMS) used in the first negative mold was Elastosil RT-607 from WACKER CHEMIE. Two components (A, B) were mixed in a ratio of 9:1, casted into the mold and hardened for 30 minutes at 80° C. The second negative mold was milled out of PTFE. Fig. 3 shows the first (1) and second (2) negative mold stacked on top of each other.

To manufacture the wax capsules, paraffin wax from ALDRICH (CAS: 8002-74-2) with a melting temperature of 53° to 57° C was used. For this purpose, molten paraffin wax was casted into the silicone mold. In order to adequately shape the recesses of the hemispheres, the PTFE mold was put over the silicone mold and pressed down firmly. Guiding holes (3) and guiding bars prevented an improper positioning. After the paraffin wax had hardened, the molds were disassembled and the wax capsule hemispheres were withdrawn.

To assemble the wax capsule hemispheres a hot air blower was used in order to ensure that the edges of the hemispheres were partially melted. As long as the edges are molten the corresponding hemispheres can be attached and pressed against each other. The assembled hemispheres form a wax capsule which can be filled with the at least one microorganism or nucleic acid, for example by injection using the filling hole. The hole can be afterwards securely sealed with a wax drop.

## Claims

1. A diagnostic reagent for methods of nucleic acid preparation and/or nucleic acid detection comprising at least one microorganism and/or purified nucleic acid, which is/are embedded in a substance, preferably a waxy substance, wherein said diagnostic reagent is adapted to release the embedded microorganism and/or nucleic acid depending on the surrounding temperature.

2. The diagnostic reagent according to the preceding claim, wherein the diagnostic reagent is provided as a solid or at least on the surface solid composition under standard conditions.

3. The diagnostic reagent according to any of the preceding claims, further comprising an emulsifier.

4. The diagnostic reagent according to any of the preceding claims, further comprising at least one excipient selected from the group consisting of binders and/or fillers.

5. The diagnostic reagent according to any of the preceding claims, further comprising at least one buffer substance, detergent, chelating agent, dye, and/or enzyme.

6. The diagnostic reagent according to any of the preceding claims, wherein the microorganism and/or nucleic acid comprises nucleic acid sequences found in the genetic information of pathogens.

7. A diagnostic reagent for methods of nucleic acid preparation and/or nucleic acid detection particularly according to any of claims 1-6, wherein a waxy substance is provided as a solid enclosure under standard conditions for containing at least one microorganism and/or purified nucleic acid.

8. A method for producing a diagnostic reagent according to any of claims 1-6, comprising the following steps:
a) melting a waxy substance,
b) mixing the molten waxy substance with an at least one microorganism and/or purified nucleic acid in desired amounts to produce an emulsion,
b) solidifying the emulsion under standard conditions preferably at ambient room temperature.

9. The method according to the preceding claim, wherein an emulsifier is added to produce the emulsion.

10. The method according to any of claims 8-9, wherein aliquots of the liquid emulsion are dispensed into molds to produce pellets, sticks or other shapes.

11. The use of the diagnostic reagent according to claim 1-7 as an internal standard in nucleic acid preparation and/or nucleic acid detection.

12. A method for detecting microorganisms comprising:
withdrawal of sample material,
lysis of cells present therein,
preparation of released nucleic acids,
nucleic acid amplification by PCR,
detection of amplified nucleic acids,
wherein a diagnostic reagent according to any of claims 1-7 is used as an internal standard in said method.

13. The method according to the preceding claim, wherein the diagnostic reagent is released during the lysis of the cells and/or during the preparation of released nucleic acids.

14. The use of the diagnostic reagent according to any of claims 1-7 for storing at least one microorganism and/or purified nucleic acid in a liquid suspension in biochemical methods.

15. A kit, comprising reagents and vessels required for methods of nucleic acid preparation and/or nucleic acid detection, wherein said kit comprises the diagnostic reagent according to any of claims 1-7 and optionally a microfluidic device.
